# FASCICULE DE BREVET EUROPEEN

(11) **EP 3 803 368 B1**
(45) Date de publication et mention de la délivrance du brevet: **04.05.2022**
(21) Numéro de dépôt: 19712791.3
(22) Date de dépôt: 28.03.2019
(51) Int. Cl.: C21D 1/00, G01N 27/9013

(54) **PROCÉDÉ DE MESURE EN LIGNE DU POURCENTAGE D'AUSTÉNITE DANS LES ACIERS**
VERFAHREN ZUR INLINE-MESSUNG DES ANTEILS VON AUSTENIT IN STÄHLEN
METHOD FOR IN-LINE MEASURING THE PERCENTAGE OF AUSTENITE IN STEELS

(30) Priorité: 01.06.2018 BE 201805364
(43) Date de publication de la demande: 14.04.2021
(73) Titulaire: Centre De Recherches Metallurgiques ASBL - Centrum Voor Research In De Metallurgie vzw, 1000 Bruxelles (BE)
(72) Inventeur: MONFORT, Guy, 4420 MONTEGNEE (BE); MOREAS, Geneviève, 4520 WANZE (BE); HERBIET, Olivier, 4122 PLAINEVAUX (BE)
(74) Mandataire: AWA Benelux
(86) Numéro de dépôt international: PCT/EP2019/057867
(87) Numéro de publication internationale: WO 2019/228692

(56) Documents cités:
- JP-A- H0 862 181
- JP-A- H07 190 991
- US-A- 4 740 747
- LOIS A ET AL: "Assessment of martensite content in austenitic steel specimens by eddy current testing", INSIGHT - NON-DESTRUCTIVE TESTING AND CONDITION MONITORING, BRITISH INSTITUTE OF NON-DESTRUCTIVE TESTING, GB, vol. 48, no. 1, 1 janvier 2006 (2006-01-01), pages 26-29, XP002459422, ISSN: 1354-2575, DOI: 10.1784/INSI.2006.48.1.26

## Description

### Objet de l'invention

La présente invention se rapporte à un procédé permettant de mesurer, en ligne de production, le pourcentage d'austénite présent dans les aciers au carbone et notamment dans les nouveaux aciers AHSS (Advanced High Strength Steels) destinés principalement à l'industrie automobile.

### Arrière-plan technologique et état de la technique

Lors de la production d'aciers au carbone, la connaissance de la fraction d'austénite par rapport à la matrice ferritique est importante à plusieurs niveaux du procédé de fabrication.

Un exemple concerne le laminage à chaud. Cette opération se fait généralement à des températures plus élevées que la transformation de phase ferrite - austénite pour des raisons liées à la puissance nécessaire pour le laminoir et aussi pour assurer un point de départ stable et connu aux modifications de microstructure qui définissent les propriétés finales du produit.

Après l'opération de laminage, les aciers sont généralement refroidis de manière contrôlée sur une table de sortie. Les vitesses de refroidissement imposées définissent la microstructure finale et les proportions des différentes phases (ferrite, bainite, martensite, perlite) qui constitueront le produit fini.

Pour contrôler la vitesse de refroidissement et obtenir ainsi une microstructure finale constante sur toute la bande d'acier et donc des propriétés mécaniques contrôlées, il est intéressant de mesurer la proportion d'austénite non encore transformée en fonction du temps. On peut alors agir sur cette évolution en modifiant le débit des rampes d'aspersion d'eau et ainsi changer la vitesse instantanée de refroidissement.

Pour cette application, on utilise généralement des dispositifs magnétiques qui doivent pouvoir fonctionner en présence de produits à température élevée (jusqu'à 850 °C) et réagir rapidement à des variations de microstructure.

Dans l'état actuel de la technique et à la connaissance des inventeurs, il existe peu de dispositifs qui soient utilisables dans ces conditions. De plus, ceux-ci présentent des limitations qui rendent la mesure imprécise ou encore ils sont affectés d'une sensibilité excessive aux variations des conditions de mesure (vibration, changement d'épaisseur de bande,...).

On peut citer le dispositif *PhaseTrack* développé par le centre de recherche d'ArcelorMittal à Maizières en France (référence : par exemple, https://www.tandfonline.com/doi/full/10.1179/1743281214Y.0000000183?scroll= top&needAccess=true). Cet appareil consiste principalement en un noyau métallique en forme de "E". Une bobine couplée à ce noyau produit un champ magnétique dans la bande. En mesurant le champ magnétique induit et en utilisant un modèle mathématique approprié, on peut déduire le pourcentage d'austénite contenue dans la bande au moment de la mesure. Cependant, ce dispositif possède deux inconvénients. Tout d'abord, il est sensible à la distance entre la bande et le capteur lui-même, ce qui peut influencer défavorablement la mesure en cas de vibrations ou d'usure des rouleaux de support de la bande par exemple. Ensuite, le modèle théorique sous-jacent doit être adapté en fonction de la nuance d'acier à mesurer.

Le deuxième dispositif, plus récent, s'appelle EMSpec et a été développé par l'Université de Manchester et le centre de recherche de Tata Steel aux Pays-Bas. Il est commercialisé par Primetals (http://www.ndt.net/article/wcndt2016/papers/mo1g4.pdf). La conception de ce système fait qu'il est largement insensible à la distance bande - capteur du fait qu'il se base sur la phase des champs magnétiques induits plutôt que sur leur amplitude. Néanmoins, la mesure nécessite l'utilisation d'un courant alternatif dont on fait varier la fréquence de 200 Hz à 50 kHz. Si on se réfère aux profondeurs de pénétration de tels courants dans des matériaux de perméabilités magnétiques différentes comme la ferrite et l'austénite, on constate que la mesure se fait sur des profondeurs largement différentes selon la phase concernée. La profondeur de pénétration peut valoir plusieurs millimètres pour la phase austénitique mais diminuer, aux plus hautes fréquences, à moins de 30 µm pour la phase ferritique. Ce fait peut entraîner des erreurs importantes si les proportions de phases sont différentes en extrême peau et dans la masse. Ceci est d'autant plus marqué que la taille de grain du matériau est importante et se rapproche de la profondeur de pénétration.

Une autre étape de fabrication où la mesure de la fraction d'austénite présente un grand intérêt est la sortie des fours de recuit ou des bains de galvanisation dans le cadre de la production de nouveaux aciers de résistance (AHSS). En effet, à ces endroits, ces nouveaux aciers contiennent une certaine proportion d'austénite qui va se transformer en une phase plus dure (martensite), soit au cours du refroidissement ultérieur, soit au cours de la mise en forme du matériau chez le client de la sidérurgie. La constance des propriétés mécaniques de ces aciers est conditionnée par la constance de la proportion d'austénite à un endroit donné du process. Afin de contrôler ce paramètre, il est important de pouvoir mesurer cette proportion d'austénite.

Au niveau de la sortie du bain de galvanisation et à la connaissance des inventeurs, seuls les dispositifs décrits ci-dessus seraient capables de faire cette mesure. Néanmoins, outre les inconvénients déjà mentionnés, ils ne sont pas optimisés pour des mesures à cet endroit et notamment la distance bande - capteur doit être plus grande que sur une table de sortie pour des raisons liées aux vibrations possibles de la bande. Cela réduit la sensibilité de la mesure et les vibrations sont également défavorables au fonctionnement de l'un de ces systèmes de mesure.

En sortie de recuit, on peut se placer à des températures plus basses auxquelles d'autres dispositifs peuvent fonctionner. Il s'agit par exemple de l'appareil 3MA développé par l'Institut Fraunhofer en Allemagne (http://www.qnetworld.com/pdfs/3MA-II datasheet web.pdf). Ce système permet la mesure de 22 paramètres magnétiques caractérisant les propriétés de l'acier. Même si ce système est essentiellement prévu pour des mesures off-line, une implémentation sur ligne de production semble possible. Cependant, ce système nécessite un étalonnage complexe qui doit être refait dès que la nuance d'acier change. De plus, il est également sensible à la distance bande - capteur et aux vibrations.

Un autre dispositif développé par la firme EMG, l'IMPOC (http://www.emg-automation.com/en/automation/quality-assurance-systems/emg-impoc/) est spécifiquement conçu pour effectuer des mesures en ligne de production. Le principe consiste à générer un champ magnétique induit sur chaque face de la bande et à mesurer le gradient ainsi obtenu. A partir de lois de corrélations, on peut déduire les propriétés mécaniques de la tôle et, par un étalonnage approprié, la proportion d'austénite. Cependant, outre que le système ne peut travailler qu'à des températures proches de la température ambiante, il est aussi sensible à la distance bande - capteur, même si l'utilisation de mesures moyennes permet de s'affranchir de l'effet des vibrations.

Dans le document JP H07 190991 A, on vise à obtenir un procédé et un dispositif permettant de mesurer le taux de transformation d'une plaque d'acier dont le rapport S/B est amélioré en éliminant le bruit dû à la rotation des rouleaux par suppression des fluctuations dans la mesure de densité de flux magnétique créé par un moyen de génération de flux magnétique, ces fluctuations étant générées en fonction d'un changement de la température ambiante, où le dispositif de mesure du taux de transformation est installé et en fonction de l'écoulement du temps.

Le dispositif comprend un boîtier incluant un équipement de magnétisation destiné à générer un flux magnétique à partir du côté inférieur d'une plaque d'acier, un boîtier de détection comprenant un capteur magnétique destiné à détecter le flux magnétique sur le côté supérieur de la plaque d'acier et un circuit de traitement de signal destiné à obtenir la valeur mesurée du taux de transformation à partir de la mesure du capteur magnétique, l'équipement de magnétisation étant installé à un point de mesure A et à un point de référence B juste avant un enrouleur sur une même ligne de fabrication. Ensuite, avec une valeur de mesure au point de mesure B comme valeur de référence correspondant à un taux de transformation de 100%, on fournit un circuit de fonctionnement pour compenser et calculer une valeur mesurée du taux de transformation au point de mesure A sur la base de la valeur de référence. Les documents JP H08 62181 A et JP H07 325067 A décrivent des installations et des méthodes de mesure très similaires.

Le document US 4,740,747 divulgue un procédé et un appareil pour mesurer le degré de transformation de la structure d'un objet. Un objet est placé entre une bobine de transmission et une bobine de réception. Un courant alternatif ou un courant d'impulsion ou une combinaison du courant alternatif et du courant d'impulsion sont fournis à la bobine de transmission, et le degré de transformation est détecté au moyen d'un signal électrique obtenu à travers la bobine de réception.

Dans A. LOIS et al., « Assessment of martensite content in austenic stainless steel specimens by eddy current testing », in Insight - Non-Destructive Testing and Condition Monitoring (BINDT), Vol. 48(1), Jan. 2006, pp. 26-29, on applique un test par courants de Foucault (ECT) pour estimer la teneur en martensite dans un acier inoxydable austénitique à partir de la modélisation d'impédance (Z) de sondes couplées à des matériaux légèrement magnétiques, et grâce à un étalonnage adéquat. Les tensions mesurées pendant les tests ont été mathématiquement transformées dans le plan d'impédance et ont été comparées aux courbes théoriques Z, montrant une bonne correspondance. Une relation linéaire entre les composantes expérimentales de Z et la teneur en alpha des échantillons a été vérifiée, ce qui rend cette procédure appropriée pour l'évaluation de la teneur en martensite dans ce type d'aciers inoxydables si des pièces d'étalonnage adéquates sont disponibles.

### Buts de l'invention

La présente invention vise à s'affranchir des limitations des dispositifs de mesure actuels en ligne de production de la fraction d'austénite dans les aciers au carbone.

En particulier, la présente invention selon le procédé de la revendication indépendante 1 a pour but de permettre la mesure pratiquement en temps réel tant à haute qu'à basse température de la bande d'acier.

Elle a aussi pour but de permettre la mesure à une distance de plusieurs dizaines de millimètres de la bande en gardant une sensibilité suffisante.

Un autre but de l'invention est de permette une mesure qui n'est pas influencée par les vibrations de la tôle ainsi que par des changements de la distance capteur - bande pouvant être relativement importants.

Enfin procédé selon l'invention doit être simple, bon marché et fonctionner au-dessus comme en dessous du point de Curie.

### Principaux éléments caractéristiques de l'invention

L'appareil utilisé dans le procédé de la présente invention se caractérise par deux bobines constituées de fil conducteur de l'électricité, par exemple de cuivre, enroulé autour d'un noyau ferromagnétique.

La première bobine, dite bobine émettrice, est raccordée à un générateur de courant alternatif, la seconde bobine, dite bobine réceptrice, est raccordée à un appareil qui permet de mesurer la tension alternative générée à ses bornes, par exemple un voltmètre mais aussi tout autre dispositif tel que système d'acquisition, d'enregistrement et/ou de visualisation.

Les deux bobines sont disposées de part et d'autre de la bande d'acier avec une distance fixe entre elles, la distance entre la bande et chacune des bobines pouvant par contre varier suite, par exemple, aux vibrations de la bande.

Plus précisément, un premier aspect de la présente invention se rapporte à un procédé selon la revendication indépendante 1 pour la mesure électromagnétique et en temps réel du pourcentage d'austénite contenu dans une bande d'acier en défilement continu lors d'un procédé de fabrication ou de transformation en ligne de celle-ci, au moyen d'un dispositif constitué de ladite bande d'acier et d'un appareil de mesure comprenant au moins :
- un générateur de courant alternatif ;
- une première bobine alimentée par le générateur de courant alternatif, dite bobine émettrice et une seconde bobine, dite bobine réceptrice, la première et seconde bobines étant disposées parallèles entre elle ou coaxiales et de part et d'autre de la bande d'acier, la distance entre les bobines étant fixe et comprise entre 10 et 200mm ;
- un noyau en matériau ferromagnétique constituant respectivement le centre de chaque bobine ;
- au moins un appareil de mesure de tension connecté aux bornes de la bobine réceptrice, sous forme d'un multimètre ou d'un système d'acquisition électronique comprenant un convertisseur analogue-digital couplé à un ordinateur, pour obtenir le pourcentage d'austénite contenu dans la bande d'acier après calibration préalable de l'appareil ;
- des moyens de calibration préalable de l'appareil ;
   de sorte que le champ magnétique produit par le courant alternatif circulant dans la bobine émettrice produit dans la bande d'acier des courants induits qui génèrent un champ magnétique induit créant dans la bobine réceptrice une force électromotrice Vs mesurable par l'appareil de mesure de tension, l'amplitude de cette force électromotrice étant fonction de la tension Vp appliquée à la bobine émettrice et de la nature de l'acier de la bande ;
ledit procédé étant caractérisé au moins par les étapes suivantes :
- on met en oeuvre un système de refroidissement des bobines pour porter celles-ci à température ambiante ;
- on fait défiler une bande d'acier de fraction austénitique inconnue entre les bobines et on mesure la tension Vs générée aux bornes de la bobine réceptrice pour une tension donnée Vp appliquée à la bobine émettrice dans une plage de tensions où les rapports entre les tensions Vs et Vp restent constants si la tension dans la bobine émettrice Vp est modifiée ;
- en tenant compte d'une calibration préalable de l'appareil, on détermine la fraction austénitique de la bande d'acier en fonction de la tension générée Vs.

Selon des formes d'exécution préférées de l'invention, le procédé est en outre limité par une des caractéristiques suivantes ou une combinaison appropriée d'entre elles :
- la calibration préalable de l'appareil est effectuée en faisant défiler entre les deux bobines des bandes de différentes nuances d'acier et de fraction austénitique connue, et en mesurant la tension générée aux bornes de la bobine réceptrice, pour chaque valeur de tension à la bobine émettrice se trouvant dans une certaine gamme ;
- la proportion d'austénite dans la bande d'acier est contrôlée lors du procédé de fabrication ou de transformation en ligne de celle-ci, de manière à assurer la constance de la phase austénitique et par suite, la constance des propriétés mécaniques de l'acier avant transformation ultérieure en phases de finition plus dures ;
- dans le dispositif utilisé, le noyau en matériau ferromagnétique comprend au moins une plaque plane mince parallèle à la surface de la bande d'acier ;
- dans le dispositif utilisé, le noyau en matériau ferromagnétique comprend au moins un cylindre d'axe parallèle ou perpendiculaire à la surface de la bande d'acier ;
- dans le dispositif utilisé, les deux bobines sont de même taille et sont placées de manière symétrique par rapport à la bande d'acier ;
- dans le dispositif utilisé, la fréquence du générateur de courant alternatif est comprise entre 50 et 500Hz ;
- dans le dispositif utilisé, la fréquence du générateur de courant alternatif est comprise entre 50 et 60Hz ;
- dans le dispositif utilisé, le noyau en matériau ferromagnétique comprend au moins une plaque principale ou un cylindre et un barreau ou une plaque secondaire perpendiculaire à la plaque principale ou au cylindre à chaque extrémité de celle-ci ou celui-ci, pour courber les lignes de champ qui seront générées ou captées par les bobines respectives ;
- dans le dispositif utilisé, l'axe des bobines est perpendiculaire à la direction de défilement de la bande ;
- dans le dispositif utilisé, la distance fixe entre les bobines est comprise entre 10 et 20 mm ;
- dans le dispositif utilisé, chaque bobine comprend de 100 à 500 spires de fil électrique apte à laisser passer un courant alternatif compris entre 2 et 5A ;
- dans le dispositif utilisé, le nombre de spires est de 5 à 10 fois plus grand dans la bobine réceptrice que dans la bobine émettrice ;
- dans le dispositif utilisé, la taille ou section de bande couverte par la plaque mince ou le cylindre est de l'ordre de 100x100mm à 200x200mm ;
- dans le dispositif utilisé, le générateur de courant alternatif est un autotransformateur rhéostat ;
- le dispositif utilisé comporte un système de refroidissement à eau ou à air pour maintenir les bobines à une température proche de la température ambiante, de préférence de manière régulée.

Un second aspect de la présente invention concerne une utilisation du procédé précité dans un laminoir à chaud selon la revendication 17, dans le cas d'une bande d'acier dont la température est inférieure à la température de Curie, la fraction austénitique de la bande d'acier étant déterminée en utilisant des fluctuations de perméabilité magnétique.

Un troisième aspect de la présente invention se rapporte à une utilisation du procédé précité dans un laminoir à chaud selon la revendication dans le cas d'une bande d'acier dont la température est supérieure à la température de Curie, la fraction austénitique de la bande d'acier étant déterminée en utilisant des fluctuations de courants de Foucault liées à la différence de résistivité entre l'austénite et la ferrite.

### Brève description des figures

La figure 1 décrit schématiquement le principe du dispositif, qui ne fait pas partie de l'invention.
La figure 2 illustre l'évolution, à température ambiante, de la tension aux bornes de la bobine réceptrice en fonction de la tension injectée aux bornes de la bobine émettrice pour divers types d'aciers contenant différentes fractions d'austénite.

### Description détaillée de l'invention

L'invention se rapporte à un procédé permettant de mesurer la proportion d'austénite dans un acier au carbone. Cette invention vise essentiellement, mais pas exclusivement, deux applications dans la production d'aciers :
- le contrôle du refroidissement sur la table de sortie au laminage à chaud où la connaissance du pourcentage d'austénite permet de contrôler les vitesses de refroidissement ;
- le contrôle des propriétés mécaniques des aciers AHSS à la sortie des lignes de recuit et des bains de galvanisation.

Dans ces deux cas, le contrôle de la proportion d'austénite permet d'assurer la constance des propriétés mécaniques en assurant la constance de la phase austénitique qui doit se transformer en phases plus dures pendant la fin du refroidissement en ligne ou pendant la déformation de l'acier chez le client.

Le dispositif employé dans le procédé selon l'invention est constitué principalement de deux bobines, elles-mêmes fabriquées chacune en enroulant un fil conducteur électrique sur un noyau ferromagnétique. Ce noyau est généralement constitué d'une plaque, pouvant être plate ou cintrée, de quelques millimètres d'épaisseur et éventuellement terminée, à chaque extrémité, par un barreau ou une autre plaque perpendiculaire à la plaque principale. Ces extrémités, qui ne sont pas indispensables à l'invention, ont pour but de courber les lignes du champ magnétique qui sera généré ou capté par les bobines.

Les dimensions de la plaque principale du noyau peuvent varier dans une large mesure selon qu'on veut obtenir une valeur moyenne du pourcentage d'austénite sur toute la largeur de la bande d'acier ou plus localement afin d'établir éventuellement un profil de distribution de la fraction austénitique sur la largeur de la bande.

Les plaques sont préférentiellement placées parallèlement à la surface de la tôle, une de chaque côté, en face l'une de l'autre, et le fil est bobiné préférentiellement dans le sens de la largeur de la bande d'acier bien que toute autre configuration puisse être envisagée. Par exemple, les plaques pourraient avantageusement être remplacées par des cylindres d'axe placé soit parallèlement soit perpendiculairement à la surface de la bande d'acier, un de chaque côté de celle-ci.

Un générateur de courant fait passer un courant alternatif dans la première bobine. Ce courant variable au cours du temps génère un champ magnétique variable dans la bande d'acier avec naissance de courants induits, les courants de Foucault. Selon la nature plus ou moins ferromagnétique et plus ou moins résistive de cette bande, ces courants de Foucault créent un champ magnétique induit plus ou moins important qui traverse et est capté par la deuxième bobine (bobine réceptrice). Ce champ génère, aux bornes de cette bobine, une tension (force électromotrice) alternative dont l'amplitude dépend de la nature du matériau constituant la bande d'acier. Cette tension peut être mesurée ou enregistrée à l'aide d'un dispositif tel qu'un voltmètre ou de tout autre système d'acquisition ou de mesure dont le principe est connu de l'homme de l'art.

Par un étalonnage simple et direct, il est alors facile de relier la tension mesurée au pourcentage d'austénite présent dans la bande d'acier.

L'inductance de la bobine réceptrice est conditionnée par la sensibilité du dispositif de mesure. Il est ainsi possible d'utiliser par exemple une centaine de spires pour la première bobine et un courant de quelques ampères tout en utilisant un nombre de spires cinq à dix fois plus grand pour la bobine secondaire.

La fréquence du courant alternatif est idéalement assez basse de manière à éviter l'effet pelliculaire et à permettre une analyse dans la plus grande épaisseur possible de la tôle. Une fréquence de 50 à 60Hz est particulièrement intéressante puisqu'elle correspond à celle des réseaux de distribution d'énergie électrique.

Une fréquence plus basse n'est pas conseillée car le temps de réaction pour stabiliser la mesure deviendrait trop important pour les vitesses de production des lignes industrielles visées principalement par l'invention.

A l'inverse, on peut utiliser une fréquence plus élevée, jusqu'à 500Hz environ, de manière à garder une profondeur de pénétration des courants induits réduite mais suffisante.

Ce dispositif est particulièrement simple et peut être fabriqué en matériaux résistant à la température. Un système de refroidissement par eau ou par air par exemple permet de l'utiliser à des endroits ou la bande à mesurer est à température élevée.

De plus, comme l'intensité de l'induction magnétique varie linéairement avec la distance, seule la distance entre les deux bobines doit être constante et non la distance entre une quelconque des bobines et la tôle. Ceci permet de s'affranchir de vibrations ou de changements de position de la bande à mesurer.

Il en résulte que la présente invention permet d'éviter la majorité des inconvénients des systèmes de mesure décrits dans l'état de l'art.

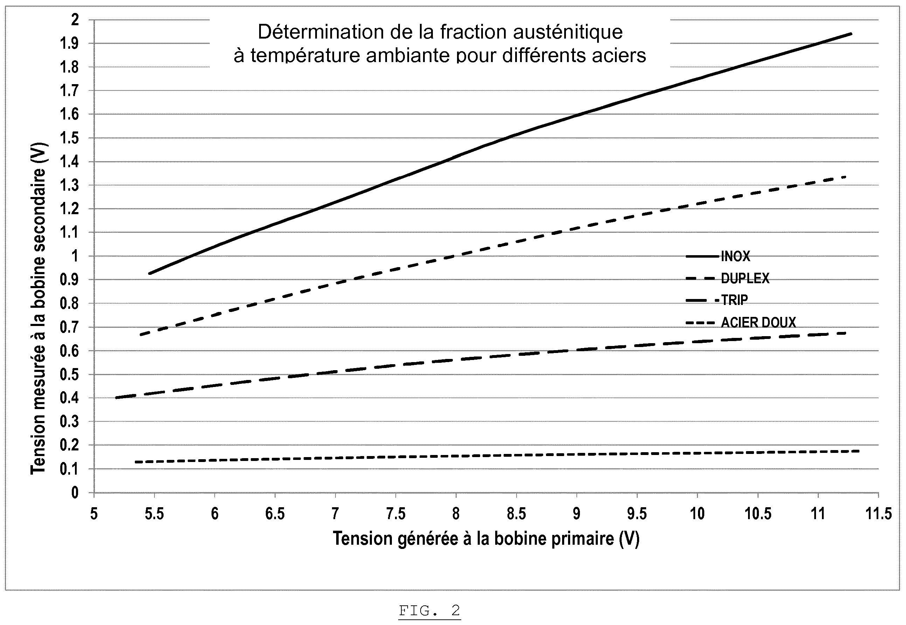

La figure 2 illustre la sensibilité de la mesure à température ambiante sur quatre types d'acier qui contiennent des pourcentages différents d'austénite, depuis un acier purement ferritique ("Acier doux") jusqu'à un acier purement austénitique ("Inox"). Les aciers "TRIP" et "Duplex" contiennent respectivement environ 15 - 20% et 60% d'austénite. En appliquant une tension à la bobine émettrice (abscisse), on génère une tension dans la bobine réceptrice (ordonnée) qui dépend du pourcentage d'austénite dans l'acier. De plus, comme on le constate sur cette figure, les rapports entre les tensions restent pratiquement constants si la tension dans la bobine émettrice est modifiée.

Dans le document JP H07 190991 A, le système de détection est destiné aux tables de sortie des laminoirs à chaud et pour une utilisation correspondant à des changements de phase se produisant à des températures inférieures à la température de Curie parce qu'il se base exclusivement sur la corrélation entre les variations de perméabilité magnétique et les caractéristiques mécaniques de la bande d'acier.

Dans la présente demande, le système de détection est utilisable également au-dessus du point de Curie parce que le traitement du signal inclut les variations dues aux courants de Foucault liées à la résistivité différente des phases austénitiques et ferritiques. Cette propriété est intéressante car s'il existe des aciers se trouvant dans une phase 100% austénitique dont la température est inférieure à la température de Curie (les aciers eutectoïdes par exemple), pour la plupart des aciers et notamment les nouveaux aciers AHSS, la transformation de phase s'effectue au-dessus de la température de Curie.

### Description d'une forme d'exécution préférée de l'invention

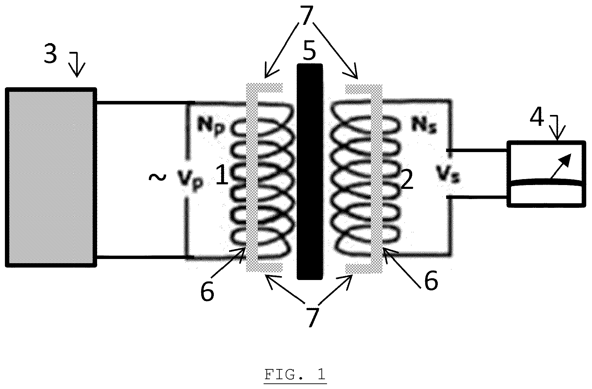

Le dispositif utilisé dans le procédé selon l'invention est décrit schématiquement à la figure 1. Les deux bobines 1, 2 sont placées de part et d'autre de la bande à analyser 5 à une distance comprise préférentiellement entre 10 et 20mm. Les plaques constituant le noyau des bobines comportent chacune une plaque principale 6 et un barreau ou une plaque secondaire 7 situé(e) perpendiculairement aux extrémités de la plaque principale 6. La taille des plaques en matériau ferromagnétique constituant le coeur des bobines varie idéalement, mais pas exclusivement, avec une section ou une surface de bande couverte de l'ordre de 100mm × 100mm jusqu'à 200mm × 200mm selon la largeur sur laquelle on veut moyenner le signal. Leur épaisseur est comprise idéalement entre 2 et 5mm.

Chaque bobine est constituée de 100 à 500 spires d'un fil de diamètre suffisant pour laisser passer un courant alternatif de l'ordre de 2 à 5 ampères obtenu par exemple à l'aide d'un autotransformateur rhéostat 3 branché sur le secteur. La tension au secondaire est mesurée à l'aide d'un multimètre 4.

L'autotransformateur est remplacé par un générateur de courant alternatif ayant une fréquence comprise entre 100 et 500Hz, ce qui permet de garder une profondeur de pénétration suffisante pour l'analyse de tôles minces (1 à 2mm d'épaisseur) tout en permettant de réagir plus rapidement à des variations locales du pourcentage d'austénite.

Le dispositif comprend en plus un circuit de refroidissement, idéalement par eau ou par un autre fluide caloporteur. Celui-ci maintient la température des bobines à une valeur proche de la température ambiante, de préférence de manière régulée. Cette forme d'exécution permet d'effectuer des mesures sur une bande d'acier portée à haute température sans augmenter de manière incontrôlée la résistivité du fil des bobines.

Le multimètre est remplacé par un système d'acquisition électronique constitué d'un convertisseur analogique-digital connecté à un ordinateur qui enregistre et visualise les valeurs mesurées. Cet ordinateur peut également convertir la tension mesurée en pourcentage d'austénite grâce à l'utilisation de données d'étalonnage établies au préalable.

## Revendications

1. Procédé de mesure électromagnétique et en temps réel du pourcentage d'austénite contenu dans une bande d'acier (5) en défilement continu lors de la fabrication ou de transformation en ligne de celle-ci, au moyen d'un dispositif constitué de ladite bande d'acier (5) et d'un appareil de mesure comprenant au moins :
- un générateur de courant alternatif (3) ;
- une première bobine alimentée par le générateur de courant alternatif (3), dite bobine émettrice (1) et une seconde bobine dite bobine réceptrice (2), la première et seconde bobines (1, 2) étant disposées parallèles entre elles ou coaxiales et de part et d'autre de la bande d'acier (5), la distance entre les bobines étant fixe et comprise entre 10 et 200mm;
- un noyau en matériau ferromagnétique (6, 7) constituant respectivement le centre de chaque bobine (1, 2) ;
- au moins un appareil de mesure de tension (4) connecté aux bornes de la bobine réceptrice (2), sous forme d'un multimètre ou d'un système d'acquisition électronique comprenant un convertisseur analogue-digital couplé à un ordinateur; pour obtenir le pourcentage d'austénite contenu dans la bande d'acier (5) après calibration préalable de l'appareil ;
- des moyens de calibration préalable de l'appareil ;
de sorte que le champ magnétique produit par le courant alternatif circulant dans la bobine émettrice (1) produit dans la bande d'acier (5) des courants induits qui génèrent un champ magnétique induit créant dans la bobine réceptrice (2) une force électromotrice Vs mesurable par l'appareil de mesure de tension (4), l'amplitude de cette force électromotrice étant fonction de la tension Vp appliquée à la bobine émettrice (1) et de la nature de l'acier de la bande (5) ;
ledit procédé étant caractérisé au moins par les étapes suivantes :
- on met en oeuvre un système de refroidissement des bobines (1, 2) pour porter celles-ci à température ambiante ;
- on fait défiler une bande d'acier de fraction austénitique inconnue entre les bobines (1, 2) et on mesure la tension Vs générée aux bornes de la bobine réceptrice (2) pour une tension donnée Vp appliquée à la bobine émettrice (1), dans une plage de tensions où les rapports entre les tensions Vs et Vp restent constants si la tension dans la bobine émettrice Vp est modifiée ;
- en tenant compte d'une calibration préalable de l'appareil, on détermine la fraction austénitique de la bande d'acier en fonction de la tension générée Vs.

2. Procédé selon la revendication 1, **caractérisé en ce que** la calibration préalable de l'appareil est effectuée en faisant défiler entre les deux bobines (1, 2) des bandes de différentes nuances d'acier et de fraction austénitique connue et en mesurant la tension générée aux bornes de la bobine réceptrice (2), pour chaque valeur de tension à la bobine émettrice (1) se trouvant dans une certaine gamme.

3. Procédé selon la revendication 1 ou 2, **caractérisé en ce que** la proportion d'austénite dans la bande d'acier (5) est contrôlée lors du procédé de fabrication ou de transformation en ligne de celle-ci, de manière à assurer la constance de la phase austénitique et par suite la constance des propriétés mécaniques de l'acier avant transformation ultérieure en phases de finition plus dures.

4. Procédé selon la revendication 1 ou 2, **caractérisé en ce que**, dans le dispositif utilisé, le noyau en matériau ferromagnétique comprend au moins une plaque plane mince (6) parallèle à la surface de la bande d'acier.

5. Procédé selon la revendication 1 ou 2, **caractérisé en ce que**, dans le dispositif utilisé, le noyau en matériau ferromagnétique comprend au moins un cylindre (6) d'axe parallèle ou perpendiculaire à la surface de la bande d'acier.

6. Procédé selon la revendication 1 ou 2, **caractérisé en ce que**, dans le dispositif utilisé, les deux bobines (1, 2) sont de même taille et sont placées de manière symétrique par rapport à la bande d'acier.

7. Procédé selon la revendication 1 ou 2, **caractérisé en ce que**, dans le dispositif utilisé, la fréquence du générateur de courant alternatif (3) est comprise entre 50 et 500Hz.

8. Procédé selon la revendication 1 ou 2, **caractérisé en ce que**, dans le dispositif utilisé, la fréquence du générateur de courant alternatif (3) est comprise entre 50 et 60Hz.

9. Procédé selon la revendication 4 ou 5, **caractérisé en ce que**, dans le dispositif utilisé, le noyau en matériau ferromagnétique comprend au moins une plaque principale ou un cylindre (6) et un barreau ou une plaque secondaire (7) perpendiculaire à la plaque principale ou au cylindre à chaque extrémité de celle-ci ou celui-ci, pour courber les lignes de champ qui seront générées ou captées par les bobines respectives.

10. Procédé selon la revendication 1 ou 2, **caractérisé en ce que**, dans le dispositif utilisé, l'axe des bobines (1, 2) est perpendiculaire à la direction de défilement de la bande.

11. Procédé selon la revendication 1 ou 2, **caractérisé en ce que**, dans le dispositif utilisé, la distance fixe entre les bobines est comprise entre 10 et 20mm.

12. Procédé selon la revendication 1 ou 2, **caractérisé en ce que**, dans le dispositif utilisé, chaque bobine (1, 2) comprend de 100 à 500 spires de fil électrique apte à laisser passer un courant alternatif compris entre 2 et 5A.

13. Procédé selon la revendication 12, **caractérisé en ce que**, dans le dispositif utilisé, le nombre de spires est de 5 à 10 fois plus grand dans la bobine réceptrice (2) que dans la bobine émettrice (1).

14. Procédé selon la revendication 4 ou 5, **caractérisé en ce que**, dans le dispositif utilisé, la taille ou section de bande couverte par la plaque mince ou le cylindre est de l'ordre de 100x100mm à 200x200mm.

15. Procédé selon la revendication 1 ou 2, **caractérisé en ce que**, dans le dispositif utilisé, le générateur de courant alternatif (3) est un autotransformateur rhéostat.

16. Procédé selon la revendication 1 ou 2, **caractérisé en ce que** le dispositif utilisé comporte un système de refroidissement à eau ou à air pour maintenir les bobines (1, 2) à une température proche de la température ambiante, de préférence de manière régulée.

17. Utilisation du procédé selon la revendication 1 ou 2 dans un laminoir à chaud, dans le cas d'une bande d'acier dont la température est inférieure à la température de Curie, la fraction austénitique de la bande d'acier étant déterminée en utilisant des fluctuations de perméabilité magnétique.

18. Utilisation du procédé selon la revendication 1 ou 2 dans un laminoir à chaud, dans le cas d'une bande d'acier dont la température est supérieure à la température de Curie, la fraction austénitique de la bande d'acier étant déterminée en utilisant des fluctuations de courants de Foucault liées à la différence de résistivité entre l'austénite et la ferrite.

## Patentansprüche

1. Verfahren zur elektromagnetischen und Echtzeitmessung des Anteils von Austenit, enthalten in einem Stahlband (5) in kontinuierlichem Rollen bei der Inline-Herstellung oder -Umformung desselben mit Hilfe einer Vorrichtung, die aus dem Stahlband (5) und einem Messgerät besteht, umfassend mindestens:
- einen Wechselstromgenerator (3),
- eine erste Spule, die vom Wechselstromgenerator (3) versorgt wird, bezeichnet als Sendespule (1), und eine zweite Spule, bezeichnet als Aufnahmespule (2), wobei die erste und zweite Spule (1, 2) parallel zueinander oder koaxial und auf beiden Seiten des Stahlbands (5) angeordnet sind, wobei der Abstand zwischen den Spulen fest ist und im Bereich zwischen 10 und 200 mm liegt;
- einen Kern aus ferromagnetischem Material (6, 7), der jeweils das Zentrum jeder Spule (1, 2) darstellt;
- mindestens ein Gerät zur Messung der Spannung (4), das mit den Klemmen der Aufnahmespule (2) verbunden ist, in Form eines Multimeters oder eines Systems zur elektronischen Erfassung, umfassend einen Analog-Digital-Wandler, der mit einem Computer gekoppelt ist, um den Anteil von Austenit, der in dem Stahlband (5) enthalten ist, nach der vorherigen Kalibrierung des Geräts zu erhalten;
- Mittel zur vorherigen Kalibrierung des Geräts;
- so dass das Magnetfeld, erzeugt durch den Wechselstrom, der in der Sendespule (1) zirkuliert, im Stahlband (5) induzierte Ströme erzeugt, die ein induziertes Magnetfeld generieren, das in der Aufnahmespule (2) eine elektromotorische Kraft Vs erzeugt, die durch das Gerät zur Messung der Spannung (4) gemessen werden kann, wobei die Amplitude dieser elektromotorischen Spannung eine Funktion der Spannung Vp, angewendet auf die Sendespule (1), und der Art des Stahlbands (5) ist;
wobei das Verfahren mindestens durch die folgenden Schritte gekennzeichnet ist:
- Implementieren eines Systems zum Abkühlen der Spulen (1, 2), um diese auf Raumtemperatur zu bringen;
- Rollenlassen eines Stahlbands mit unbekannter Austenitfraktion zwischen den Spulen (1, 2) und Messen der Spannung Vs, die an Klemmen der Aufnahmespule (2) generiert wird, für eine gegebene Spannung Vp, angewendet auf die Sendespule (1), in einem Spannungsbereich, in dem die Bezüge zwischen den Spannungen Vs und Vp konstant bleiben, wenn die Spannung in der Sendespule Vp modifiziert wird;
- unter Berücksichtigung einer vorherigen Kalibrierung des Geräts Bestimmen der austenitischen Fraktion des Stahlbands in Funktion der generierten Spannung Vs.

2. Verfahren nach Anspruch 1, **dadurch gekennzeichnet, dass** die vorherige Kalibrierung der Geräts durch Rollenlassen zwischen den zwei Spulen (1, 2) der Bänder aus verschiedenen Stahlsorten und bekannter Austenitfraktion und durch Messen der Spannung, die an Klemmen der Aufnahmespule (2) generiert wird, für jeden Wert der Spannung an der Sendespule (1), die sich in einem bestimmten Bereich befindet, durchgeführt wird.

3. Verfahren nach Anspruch 1 oder 2, **dadurch gekennzeichnet, dass** der Anteil von Austenit im Stahlband (5) beim Verfahren der Inline-Herstellung oder - Umwandlung derselben gesteuert wird, um die Konstanz der austenitischen Phase und folglich die Konstanz der mechanischen Eigenschaften des Stahls vor der endgültigen Umwandlung in härtere Verarbeitungen sicherzustellen.

4. Verfahren nach Anspruch 1 oder 2, **dadurch gekennzeichnet, dass** in der verwendeten Vorrichtung der Kern aus ferromagnetischem Material mindestens eine dünne obere Platte (6) parallel zur Oberfläche des Stahlbands umfasst.

5. Verfahren nach Anspruch 1 oder 2, **dadurch gekennzeichnet, dass** in der verwendeten Vorrichtung der Kern aus ferromagnetischem Material mindestens einen Zylinder (6) mit paralleler oder senkrechter Achse zur Oberfläche des Stahlbands umfasst.

6. Verfahren nach Anspruch 1 oder 2, **dadurch gekennzeichnet, dass** in der verwendeten Vorrichtung die zwei Spulen (1, 2) die gleiche Größe aufweisen und auf symmetrische Weise mit Bezug auf das Stahlband platziert sind.

7. Verfahren nach Anspruch 1 oder 2, **dadurch gekennzeichnet, dass** in der verwendeten Vorrichtung die Frequenz des Wechselstromgenerators (3) im Bereich zwischen 50 und 500 Hz liegt.

8. Verfahren nach Anspruch 1 oder 2, **dadurch gekennzeichnet, dass** in der verwendeten Vorrichtung die Frequenz des Wechselstromgenerators (3) im Bereich zwischen 50 und 60 Hz liegt.

9. Verfahren nach Anspruch 4 oder 5, **dadurch gekennzeichnet, dass** in der verwendeten Vorrichtung der Kern aus ferromagnetischem Material mindestens eine Hauptplatte oder einen Zylinder (6) oder eine Stange oder eine sekundäre Platte (7) senkrecht zur Hauptplatte oder zum Zylinder an jedem Ende dieser oder jenes umfasst, um die Feldlinien zu krümmen, die von den entsprechenden Spulen generiert oder erfasst werden.

10. Verfahren nach Anspruch 1 oder 2, **dadurch gekennzeichnet, dass** in der verwendeten Vorrichtung die Achse der Spulen (1, 2) senkrecht zur Rollrichtung des Bands ist.

11. Verfahren nach Anspruch 1 oder 2, **dadurch gekennzeichnet, dass** in der verwendeten Vorrichtung der feste Abstand zwischen den Spulen zwischen 10 und 20 mm liegt.

12. Verfahren nach Anspruch 1 oder 2, **dadurch gekennzeichnet, dass** in der verwendeten Vorrichtung jede Spule (1, 2) 100 bis 500 elektrische Drahtwicklungen umfasst, die ausgelegt sind, um einen Wechselstrom im Bereich zwischen 2 und 5A passieren zu lassen.

13. Verfahren nach Anspruch 12, **dadurch gekennzeichnet, dass** in der verwendeten Vorrichtung die Anzahl von Windungen 5 bis 10 Mal größer in der Aufnahmespule (2) als in der Sendespule (1) ist.

14. Verfahren nach Anspruch 4 oder 5, **dadurch gekennzeichnet, dass** in der verwendeten Vorrichtung die Größe oder der Schnitt des Bands, bedeckt von der dünnen Platte oder dem Zylinder, im Bereich von 100 × 100 mm bis 200 × 200 mm liegt.

15. Verfahren nach Anspruch 1 oder 2, **dadurch gekennzeichnet, dass** in der verwendeten Vorrichtung der Wechselstromgenerator (3) ein Rheostat-Spartransformator ist.

16. Verfahren nach Anspruch 1 oder 2, **dadurch gekennzeichnet, dass** die verwendete Vorrichtung ein System zur Kühlung mit Wasser oder mit Luft umfasst, um die Spulen (1, 2) bei einer Temperatur nahe der Raumtemperatur vorzugsweise auf geregelte Weise zu halten,

17. Verwendung des Verfahrens nach Anspruch 1 oder 2 in einem Warmwalzwerk, für den Fall eines Stahlbands, dessen Temperatur niedriger als die Curie-Temperatur ist, wobei die austenitische Fraktion des Stahlbands unter Verwendung der Fluktuationen der magnetischen Durchlässigkeit bestimmt wird.

18. Verwendung des Verfahrens nach Anspruch 1 oder 2 in einem Warmwalzwerk, für den Fall eines Stahlbands, dessen Temperatur höher als die Curie-Temperatur ist, wobei die austenitische Fraktion des Stahlbands unter Verwendung der Fluktuationen von Foucault-Strömen bestimmt wird, die mit der Widerstandsdifferenz zwischen dem Austenit und dem Ferrit verbunden ist.

## Claims

1. Method for the electromagnetic and real-time measurement of the percentage of austenite contained in steel strip (5) in continuous motion during the in-line manufacture or transformation thereof, by means of a device consisting of said steel strip (5) and of a measuring device comprising at least:
- an alternating-current generator (3);
- a first coil supplied by the alternating-current generator (3), called the transmitting coil (1), and a second coil, called the receiving coil (2), the first and second coils (1, 2) being arranged parallel to each other or coaxial and on both sides of the steel strip (5), the distance between the coils being fixed and comprised between 10 and 200mm;
- a core of ferromagnetic material (6, 7) constituting the center of each coil (1, 2), respectively;
- at least one voltage-measuring device (4) connected to the terminals of the receiving coil (2), in the form of a multimeter or of an electronic acquisition system comprising an analog-digital converter coupled to a computer, to obtain the percentage of austenite contained in the steel strip (5) after prior calibration of the device;
- means for prior calibration of the device;
so that the magnetic field produced by the alternating current flowing in the transmitting coil (1) produces in the steel strip (5) induced currents that generate an induced magnetic field creating in the receiving coil (2) an electromotive force Vs that can be measured by the voltage-measuring device (4), the amplitude of this electromotive force being a function of the voltage Vp applied to the transmitting coil (1) and of the nature of the steel of the strip (5);
said method being characterized at least by the following steps:
- a cooling system is implemented for the coils (1, 2) in order to bring them to ambient temperature;
- a steel strip of unknown austenitic fraction is scrolled between the coils (1, 2) and the voltage Vs generated at the terminals of the receiving coil (2) is measured for a given voltage Vp applied to the transmitting coil (1) in a voltage range where the ratios between the voltages Vs and Vp remain constant if the voltage in the transmitting coil Vp is modified;
- taking into account prior calibration of the device, the austenitic fraction of the steel strip is determined as a function of the generated voltage Vs.

2. Method according to Claim 1, **characterized in that** the prior calibration of the device is carried out by scrolling, between the two coils (1, 2), strips of different grades of steel and of known austenitic fraction, and by measuring the voltage generated at the terminals of the receiving coil (2), for each voltage value at the transmitting coil (1) within a certain range.

3. Method according to Claim 1 or 2, **characterized in that** the proportion of austenite in the steel strip (5) is controlled during the in-line manufacture or transformation method thereof, so as to ensure the stability of the austenitic phase and consequently the stability of the mechanical properties of the steel before subsequent transformation into harder finishing phases.

4. Method according to Claim 1 or 2, **characterized in that**, in the device used, the core of ferromagnetic material comprises at least one thin flat plate (6) that is parallel to the surface of the steel strip.

5. Method according to Claim 1 or 2, **characterized in that**, in the device used, the core of ferromagnetic material comprises at least one cylinder (6) with an axis that is parallel or perpendicular to the surface of the steel strip.

6. Method according to Claim 1 or 2, **characterized in that**, in the device used, the two coils (1, 2) are of the same size and placed symmetrically with respect to the steel strip.

7. Method according to Claim 1 or 2, **characterized in that**, in the device used, the frequency of the alternating-current generator (3) is between 50 and 500Hz.

8. Method according to Claim 1 or 2, **characterized in that**, in the device used, the frequency of the alternating-current generator (3) is between 50 and 60Hz.

9. Method according to Claim 4 or 5, **characterized in that**, in the device used, the core of ferromagnetic material comprises at least one main plate or a cylinder (6) and a bar or a secondary plate (7) that is perpendicular to the main plate or to the cylinder at each end thereof, to bend the field lines that will be generated or picked up by the respective coils.

10. Method according to Claim 1 or 2, **characterized in that**, in the device used, the axis of the coils (1, 2) is perpendicular to the motion direction of the strip.

11. Method according to Claim 1 or 2, **characterized in that**, in the device used, the fixed distance between the coils is comprised between 10 and 20mm.

12. Method according to Claim 1 or 2, **characterized in that**, in the device used, each coil (1, 2) comprises from 100 to 500 turns of electric wire that is suitable for letting an alternating current comprised between 2 and 5 A.

13. Method according to Claim 12, **characterized in that**, in the device used, the number of turns is 5 to 10 times greater in the receiving coil (2) than in the transmitting coil (1).

14. Method according to Claim 4 or 5, **characterized in that**, in the device used, the strip size or section covered by the thin plate or by the cylinder is of the order of 100×100mm to 200x200mm.

15. Method according to Claim 1 or 2, **characterized in that**, in the device used, the alternating-current generator (3) is a rheostat autotransformer.

16. Method according to Claim 1 or 2, **characterized in that** the device used comprises a water or air cooling system to maintain the coils (1, 2) at a temperature that is close to ambient temperature, preferably in a regulated manner.

17. Use of the method according to Claim 1 or 2 in a hot-rolling mill, in the case of a steel strip at a temperature that is below the Curie temperature, the austenitic fraction of the steel strip being determined by using fluctuations in magnetic permeability.

18. Use of the method according to Claim 1 or 2 in a hot-rolling mill, in the case of a steel strip at a temperature that is above the Curie temperature, the austenitic fraction of the steel strip being determined by using eddy current fluctuations linked to the difference in resistivity between austenite and ferrite.
